# EUROPEAN PATENT APPLICATION

(11) **EP 4 494 617 A1**
(43) Date of publication of application: **22.01.2025**
(21) Application number: 23186157.6
(22) Date of filing: 18.07.2023
(51) Int. Cl.: A61F 13/15, A61F 13/511, A61F 13/515, B31F 1/07

(54) **ASSORBENT ASSEMBLY**

(71) Applicant: Ontex BV, 9255 Buggenhout (BE); Ontex Group NV, 9320 Erembodegem (BE)
(72) Inventor: DESCHEEMAECKER, Evan, 9930 Zomergem (BE); ROEGIERS, Christophe, 9900 Eeklo (BE); STAELENS, Eva, 9900 Eeklo (BE)
(74) Representative: Saurat, Thibault

(57) **Abstract**

The invention pertains to an absorbent article (10) comprising :
- a liquid pervious topsheet (14) consisting essentially of natural fibers,
- a liquid impervious backsheet (16) comprising a plastic material,
- an absorbent core (12) arranged between said topsheet (14) and backsheet (16) comprising absorbent material,
wherein the topsheet (14) and the backsheet (16) are attached via attachment means to one another in a bonding region (13), said bonding region (13) being positioned along the periphery of the absorbent article (10). According to the present disclosure, said bonding region (13) is substantially free of adhesive.

## Description

### TECHNICAL FIELD

The disclosure pertains to the technical field of absorbent articles, or absorbent assemblies, for hygiene products and the packaging of such absorbent articles. In particular, the present disclosure relates to absorbent articles selected from sanitary napkins, adult incontinence pads or panty liners, which are configured to collect and contain blood, menses, urine, vaginal fluids or avoid leakage. More particularly, this invention is directed to the bonding of the topsheet and backsheet.

### BACKGROUND

Absorbent articles selected from sanitary napkins, adult incontinence pads or pantyliners, used to collect vaginal discharges are well known in the art. Typically, these absorbent articles commonly comprise a topsheet, a backsheet and an absorbent core arranged in-between said topsheet and backsheet. These articles are configured to be fastened onto an undergarment and comprise a layer of adhesive arranged on one side of the backsheet so that the article can be secured onto said garment. There is a trend to introduce more and more natural raw materials such as cotton, hemp and/or bamboo, in absorbent articles especially in feminine hygiene articles.

For example, document US20200316245 describes biodegradable absorbent articles where the article comprises a topsheet with cellulosic fibers selected from a group consisting of cotton, hemp, flax, ramie, rayon, bamboo. Document WO2020/004645 also describes absorbent articles trying to minimize chemical substances with a topsheet containing cotton fibers. However, natural fibers are not malleable and are not heat sensitive, i.e. do not melt at lower temperatures like thermoplastic material does. There is an issue to sufficiently bond said cotton-based topsheets or other natural raw material-based topsheets to standard plastic backsheets. A common approach is to use dedicated adhesives that ensure a proper bonding between natural fibers and thermoplastic fibers, however these adhesives are very expensive and not sustainable material.

Hence, there is still a need to improve the environmental impact of these absorbent articles and offer a solution that involves less unsustainable materials such as special adhesives, derived from fossil resources petrochemical reactions, without degrading the performance of these absorbent articles.

The invention thereto aims to provide an environmentally friendly solution for absorbent articles selected from diapers, training pants, incontinence pants, sanitary napkins, adult incontinence pads or pantyliners. Whilst a focus will be made on sanitary napkins and feminine hygiene, this concept can be implemented on other absorbent articles such as diapers, pants, incontinence pads, *etc.* and the present disclosure may not be limited to sanitary napkins exclusively.

### SUMMARY OF THE INVENTION

The present disclosure relates to an absorbent article comprising :
- a liquid pervious topsheet consisting essentially of natural fibers, preferably cotton, hemp, viscose or bamboo,
- a liquid impervious backsheet comprising a plastic material, preferably a laminate of polyethylene (PE) or bio-PE or low-density PE (LDPE),
- an absorbent core arranged between said topsheet and backsheet comprising absorbent material, preferably cellulose or fluff and/or superabsorbent polymers,
wherein the topsheet and the backsheet are attached via attachment means to one another in a bonding region, said bonding region being positioned along the periphery of the absorbent article. According to the present disclosure, the bonding region is substantially free of adhesive.

By the term " a liquid pervious topsheet consisting essentially of natural fibers", it is meant a topsheet consisting of a non-woven fabric or material that is formed from natural fibers, for example 100 % organic, natural cellulosic fibers. Said topsheet does not exclude the presence of additional materials which do not significantly affect the natural composition of the topsheet. For example, said topsheet can comprise skin care ingredients, perfumes or antimicrobial agents. Any number of different natural fibers can be used to form the topsheet so long as they are suitable for the intended use described herein. Exemplary natural fibers, include but are not limited to, both plant-based natural fibers and animal-based natural fibers. Plant-based natural fibers include, but are not limited to, cotton, hemp, flax, ramie, bamboo, biotic material. Cotton is the most widely used natural fiber as it is almost pure cellulose and is both soft to the touch and advantageously has breathability. As with many materials, cotton is available and marketed in an organic form which is generally understood to mean that the cotton is grown from non-genetically modified plants without the use of any synthetic agricultural chemicals, such as fertilizers or pesticides. Hemp fiber comes from the plant species cannibus sativa. Hemp is not typically certified organic, but its production is considered organic by definition. Hemp grows quickly and does not require the use of fertilizer or pesticides. Bamboo is a sustainable crop; its quick growth allows it to be replaced quickly with a new crop. Like hemp, it does not typically require fertilizers or pesticides to grow. Bamboo is nice for sanitay napkin because it is soft. Animal-based natural fibers include, but are not limited to, wool. As described herein, wool is more particularly suited as a material to form the outer shell (back sheet) since wool-based covers work very well at containing leaks and allowing air to circulate. Organic wool contains no pesticide residue and the sheep are raised organically. In addition, rayon (e.g., viscose rayon), which is a natural-based material made from the cellulose of wood pulp, is also suitable as a topsheet material.

By the term "said bonding region is substantially free of adhesive", it is meant that the adhesive bonding is not the predominant, or principal, means of attachment. There can be some minute quantities of adhesive for example should adhesive bonding the topsheet to the ADL leak to the sides and partially within the bonding regions, that quantity of adhesive would be minute or even residual. In other words, the present disclosure pertains to an absorbent article where the topsheet and backsheet are bonded primarily via bonding, or joining, techniques that are substantially adhesive-free, meaning that do not make use of adhesives as their sole, or at least main, means of attachment.

According to an embodiment, said bonding region comprises discrete attachment points or sites.

By the term "discrete attachment points or site", it is meant that the topsheet and backsheet are intermittently bonded to one another. In other words, the bonding region comprises bonded and un-bonded areas or sites, or points, where the topsheet and backsheet are respectively associated and unattached.

According to an embodiment, said discrete attachment points are arranged with a bonding density comprised between 60 and 95%, preferably between 70 and 90%.

According to an embodiment, the absorbent article further comprises an acquisition distribution layer arranged between the topsheet and the absorbent core, said acquisition distribution layer being arranged between the topsheet and the backsheet in the bonding region, the topsheet, acquisition distribution layer and the backsheet being attached altogether via said attachment means in the bonding region.

According to an embodiment, the absorbent article is a sanitary napkin.

According to an embodiment, said sanitary napkin further comprises two wings, said sanitary napkin extending in longitudinal, transversal and vertical directions, the sanitary napkin being divided with respect to the longitudinal direction, into a front portion, a rear portion and a central portion arranged between said front and rear portions, the central portion being defined by the length of the wings taken in the point in which they extend from the longitudinal sides of said sanitary napkin in a direction parallel to the longitudinal axis, wherein a layer of adhesive is arranged on the side of the backsheet opposite to the absorbent core. According to said embodiment, said layer of adhesive is arranged on the backsheet in a discontinuous pattern, said pattern of adhesive comprising: a first area of adhesive arranged in the front portion, a second area of adhesive arranged in the rear portion; a third area of adhesive being arranged on the wings; wherein said pattern of adhesive comprises an adhesive-free area between the first area of adhesive and the second area of adhesive, said adhesive-free area longitudinally extending on a length that is greater than or equal to the length of the third area of adhesive with respect to the longitudinal direction.

The present disclosure also relates to an apparatus for bonding a liquid pervious topsheet consisting essentially of natural-based material to a liquid impervious backsheet comprising a plastic material, said apparatus comprising a first bonding roller having a circumferential outer surface comprising a first flat surface and a first bonding surface, said first bonding surface comprising a plurality of grooves and ridges extending in the machine direction and/or cross direction; a second bonding roller having a circumferential outer surface comprising a second flat surface and second bonding surface, said second bonding surface comprising a plurality of protrusions, said protrusions being arranged to fit within said grooves, wherein the first and second bonding surfaces are arranged to define the periphery of at least one absorbent article and said first and second flat surfaces being arranged within the areas defined by the first and second bonding surface respectively.

According to an embodiment, the protrusions are frusto-pyramidal shaped and are aligned in the machine direction in at least one column.

According to an embodiment, said grooves and ridges are continuous in the machine direction when defining the periphery of one absorbent article.

The present disclosure also relates to a method suitable for manufacturing an absorbent article as described hereabove, the method comprising:
- providing a topsheet consisting essentially of natural fibers,
- providing a backsheet comprising plastic material,
- arranging an absorbent core comprising absorbent material in between said topsheet and backsheet,
- applying a mechanical bonding with an apparatus as described hereabove, in said bonding region along the periphery of the absorbent article to bond said topsheet to said backsheet, said bonding region being substantially free of adhesive.

According to an embodiment, the method comprises: providing a topsheet consisting essentially of natural fibers, providing a backsheet comprising plastic material,arranging an absorbent core comprising absorbent material in between said topsheet and backsheet, applying an ultrasonic bonding with an apparatus comprising: an ultrasonic welding device comprising a sonotrode inducing vibrations at a frequency between 10 kHz and 50 kHz, preferably between 15 kHz and 30 kHz, a second bonding roller having a circumferential outer surface comprising a second flat surface and second bonding surface, said second bonding surface comprising a plurality of protrusions, wherein the second bonding surface is arranged to define the periphery of at least one absorbent article and said second flat surfaces being arranged within the areas defined by the first and second bonding surface respectively, said ultrasonic bonding being applied in said bonding region along the periphery of the absorbent article to bond said topsheet to said backsheet, said bonding region being substantially free of adhesive.

All of these embodiments mentioned above can be taken individually or in combination.

Further embodiments are described below and in the claims.

### DESCRIPTION OF FIGURES

The drawings and figures are illustrative in nature and not intended to limit the subject matter defined by the claims. The following detailed description can be understood when read in conjunction with the following drawings, where like structure is indicated with like reference numerals in which:
**FIG.1** illustrates a schematic transversal cross section of an absorbent article;
**FIG.2** shows a schematic top view of an absorbent article in an unfolded configuration according to an embodiment;
**FIG.3** shows a portion of the apparatus to bond a natural material-based topsheet to a plastic material based backsheet;
**FIG.4A** is a close-up of the first bonding roller of figure FIG.3 at the zone Z102;
**FIG.4B** is a close-up of the rectangular area of FIG.4A when seen from above or in the radial direction;
**FIG.4C** is a cross section of the bonding surface of the first bonding roller,
**FIG.5A** is a close-up of the second bonding roller of figure FIG.3 at the zone Z104;
**FIG.5B** is a close-up of the rectangular area of FIG.5A when seen from above or in the radial direction;
**FIG.5C** is a cross section of the bonding surface of the second bonding roller,
**FIG.6A** illustrates the interaction between the first bonding roller and the second bonding layer at the nip when seen from above or in the radial direction;
**FIG.6B** is a cross section of the bonding surfaces of the first and second bonding rollers interacting at the nip.

### DETAILED DESCRIPTION OF THE INVENTION

The present disclosure pertains to providing an environmentally friendly solution for absorbent articles selected from diapers, training pants, incontinence pants, sanitary napkins, adult incontinence pads or pantyliners where the amount of adhesive and thus the amount of thermoplastic material is limited.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

The recitation of numerical ranges by endpoints includes all numbers and fractions subsumed within that range, as well as the recited endpoints.

As used herein, the following terms have the following meanings:
"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1 % or less of and from the specified value, in so far such variations are appropriate to perform in the disclosed disclosure. However, it is to be understood that the value to which the modifier "about" refers is itself also specifically disclosed.

"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

"Absorbent article" or "absorbent assembly", these terms being interchangeable, refers to devices that absorb and contain liquid, and more specifically, refers to devices that are placed against or in proximity to the body of the wearer to absorb and contain the various exudates discharged from the body. Absorbent articles preferably comprise a longitudinal axis defining a length and a transversal axis defining a width, said transversal axis being perpendicular to said longitudinal axis as well as a vertical axis defining a height or thickness. The longitudinal axis is hereby conventionally chosen in the front-to-back direction of the article when referring to the article being worn, and the transversal axis is conventionally chosen in the left-to-right direction of the article when referring to the article being worn. Disposable absorbent articles can include a liquid pervious topsheet, a backsheet joined to the topsheet, and an absorbent core positioned and held between the topsheet and the backsheet. The topsheet is operatively permeable to the liquids that are intended to be held or stored by the absorbent article, and the backsheet may or may not be substantially impervious or otherwise operatively impermeable to the intended liquids. The absorbent articles may also include other components, such as acquisition distribution layers, liquid intake layers, liquid distribution layers, transfer layers, barrier layers, wrapping layers and the like, as well as combinations thereof. Disposable absorbent articles and the components thereof can operate to provide a body-facing surface and a garment-facing surface.

"Front", "rear or back", and "crotch" end of the absorbent article as used herein typically refer for the "front" end of the article is that which is most proximal to the front of the subject when worn, the "rear or back" end of the article is that which is most proximal to the rear or back of the subject when worn, and the "crotch" portion of the core is the middle portion of the absorbent article between the "front" and "rear or back" portions.

The "absorbent medium" or "absorbent core" or "absorbent body" is the absorbent structure disposed between the topsheet and the backsheet of the absorbent article in at least the crotch region of the absorbent article and is capable of absorbing and retaining liquid body exudates. The size and the absorbent capacity of the absorbent medium should be compatible with the size of the intended wearer and the liquid loading imparted by the intended use of the absorbent article. Further, the size and the absorbent capacity of the absorbent medium can be varied to accommodate wearers ranging from infants through adults. It may be manufactured in a wide variety of shapes (for example, rectangular, trapezoidal, T-shape, I-shape, hourglass shape, etc.) and from a wide variety of materials. Examples of commonly occurring absorbent materials are cellulosic fluff pulp, tissue layers, highly absorbent polymers (so called superabsorbent polymer particles (SAP)), absorbent foam materials, absorbent nonwoven materials or the like. It is common to combine cellulosic fluff pulp with superabsorbent polymers in an absorbent material.

The term "adhesive" as used herein is intended to refer to any suitable hot melt, water or solvent borne adhesive that can be applied to a surface. Accordingly, adhesives include conventional hot melt adhesives, pressure-sensitive adhesives and reactive adhesives (i.e., polyurethane). The absorbent article can comprise different kinds of adhesive. For example, the absorbent article can comprise hot melt adhesive for bonding the absorbent core to the backsheet and non-structural adhesive or pressure sensitive adhesive or positioning adhesive for securing the absorbent article to an undergarment.

As used herein, the term "adhesive bonding" means a bonding process which forms a bond by application of an adhesive. Such application of adhesive may be by various processes such as slot coating, spray coating and other topical applications. Further, such adhesive may be applied within a product component and then exposed to pressure such that contact of a second product component with the adhesive containing product component forms an adhesive bond between the two components.

As used therein, the term "bonded" encompasses configurations in which topsheet is directly joined to backsheet by affixing topsheet directly to backsheet, and configurations wherein topsheet is associated or joined to backsheet by affixing topsheet to intermediate member(s) such as an acquisition distribution layer (ADL) which in turn are affixed to backsheet. Topsheet and backsheet can be affixed directly to each other by attachment means such as a ultrasonic bonds or mechanical embossment.

The term "backsheet" or "backsheet" refers to a material forming the outer cover of the absorbent article. The backsheet prevents the exudates contained in the absorbent structure from wetting articles such as undergarments which contact the disposable absorbent article. The backsheet may be a unitary layer of material or may be a composite layer composed of multiple components assembled side-by-side or laminated. The backsheet may be the same or different in different parts of the absorbent article. At least in the area of the absorbent medium the backsheet comprises a liquid impervious material in the form of a thin plastic film, e.g. a polyethylene and/or polypropylene film, a nonwoven material coated with a liquid impervious material, a hydrophobic nonwoven material, which resists liquid penetration, or a laminate of a plastic film and a nonwoven material. The backsheet material may be breathable so as to allow vapor to escape from the absorbent material, while still preventing liquids from passing through. Examples of breathable backsheet materials are porous polymeric films, nonwoven laminates of spunbond and meltblown layers and laminates of porous polymeric films and nonwoven materials.

As used herein, the term "basis weight" refers to an average weight, or a range of weight, of the component in question, namely the layer of cellulosic fibers or the layer of peeling agent, per square meter. Term such as "grammage" is equivalent to "basis weight" and is expressed in grams per square meter, g/m² or gsm.

As used herein, the term "body-facing" side or surface means that side or surface of the absorbent article or component of said absorbent article which is intended to be disposed toward or placed adjacent to the body of the wearer during ordinary use, while the "garment-facing" side or surface is on the opposite side, and is intended to be disposed to face away from the wearer's body during ordinary use. Such garment-facing surface may be arranged to face toward or placed adjacent to the wearer's undergarments when the absorbent article is worn. In other words, the "body-facing side" of a component is the side proximal to the body of the wearer whereas "garment-facing side" of a component is the side proximal to the garment or undergarment of the wearer.

"Bonded" refers to the joining, adhering, associating, connecting, attaching, or the like, of at least two elements. Two elements will be considered to be bonded together when they are bonded directly to one another or indirectly to one another, such as when each is directly bonded to intermediate elements.

As used herein, the term "cellulosic" or "cellulosic fibers" is meant to include any material having cellulose as a major constituent, and specifically comprising at least 50 percent weight cellulose or a cellulose derivative. Thus, the term includes cotton, typical wood pulps, nonwoody cellulosic fibers, kraft paper, cellulose acetate, cellulose triacetate, rayon, thermomechanical wood pulp, chemical wood pulp, debonded chemical wood pulp, milkweed, or bacterial cellulose.

The term "consisting essentially of" does not exclude the presence of additional materials which do not significantly affect the desired characteristics of a given composition or product. Exemplary materials of this sort would include, without limitation, pigments, antioxidants, skin care ingredients, odorizing agents, stabilizers, surfactants, waxes, flow promoters, solvents, particulates and materials added to enhance processability of the composition.

As used herein, the term "garment" means any type of apparel which may be worn. This includes incontinence products, surgical gowns, industrial workwear and coveralls, undergarments, underwear, pants, shirts, jackets and the like.

The term "hydrophilic" describes fibers or the surfaces of fibers which are wetted by the aqueous liquids in contact with the fibers. The degree of wetting of the materials can, in turn, be described in terms of the contact angles and the surface tensions of the liquids and materials involved. The term "wettable" is meant to refer to a fiber which exhibits a liquid, such as water, synthetic urine, or a 0.9 weight percent aqueous saline solution, in air contact angle of less than 90°, whereas "hydrophobic" or "non-wettable" describes fibers having contact angles equal to or greater than 90°.

As used herein, the term "impermeable" generally refers to articles and/or elements that are substantially not penetrated by aqueous fluid through the entire thickness thereof under a pressure of 1.0 kPa or less. Preferably, the impermeable article or element is not penetrated by aqueous fluid under pressures of 3.4 kPa or less. More preferably, the impermeable article or element is not penetrated by fluid under pressures of 6.8 kPa or less. An article or element that is not impermeable is permeable.

"Join", "joining", "joined", or variations thereof, when used in describing the relationship between two or more elements, means that the elements can be connected together in any suitable manner, such as by heat sealing, ultrasonic bonding, thermal bonding, by adhesives, stitching, or the like. Further, the elements can be joined directly together, or may have one or more elements interposed between them, all of which are connected together.

"Laminate" refers to elements being attached together in a layered arrangement.

The use of the term "layer" can refer, but is not limited, to any type of substrate, such as a woven web, nonwoven web, films, laminates, composites, elastomeric materials, cellulosic material or the like. A layer can be liquid and air permeable, permeable to air but impermeable to liquids, impermeable both to air and liquid, or the like. When used in the singular, it can have the dual meaning of a single element or a plurality of elements. In other terms, a "layer" refers to a single material or a plurality of materials forming a sheet with a given thickness and extending in length and width. The material(s) forming the layer can be arranged in a continuous or discontinuous area, region or zone.

"Liquid" means a nongaseous substance and/or material that flows and can assume the interior shape of a container into which it is poured or placed.

The term "nonwoven fabric", "nonwoven layer" or "nonwoven web" means a sheet material having a structure of individual fibers or threads which are interlaid, but not in a regular manner such as occurs with knitting or weaving processes. Nonwoven fabrics, layers or webs have been formed from many processes such as for example, meltblowing processes, spunbonding processes, and bonded carded web processes.

The term "natural fibers" means any fibers derived from a natural source or in other words, derived from plants, animals, or the ground and which is not man-made.

The term "polymer" generally includes, but is not limited to, homopolymers, copolymers, such as, for example, block, graft, random and alternating copolymers, terpolymers, etc. and blends and modifications thereof. Furthermore, unless otherwise specifically limited, the term "polymer" shall include all possible geometrical configurations of the material. These configurations include, but are not limited to, isotactic, syndiotactic and random symmetries.

"Pulp" refers to a material made up of cellulose fibers, or cellulosic fibers. The fibers can be either natural or synthetic, or a combination thereof. "Pulp fluff" or "fluff" or "fluff pulp" refers to a material made up of cellulose fibers made from softwood or hardwood, or a material comprising viscose. The material is typically lightweight and has absorbent properties.

By "substantially", it is meant at least the majority of the structure referred to.

Use of the term "substrate" includes, but is not limited to, woven or nonwoven webs, porous films, ink permeable films, cellulosic pulp, paper, composite structures, or the like.

The term "topsheet" refers to a liquid permeable material sheet forming the inner cover of the absorbent article and which in use is placed in direct contact with the skin of the wearer. The topsheet is typically employed to help isolate the wearer's skin from liquids held in the absorbent structure. Suitable nonwoven materials can be composed of man-made fibers, such as polyester, polyethylene, polypropylene, viscose, rayon etc. or natural fibers, such as wood pulp or cotton fibers, or from a mixture of natural and man-made fibers. The topsheet material may further be composed of two fibers, which may be bonded to each other in a bonding pattern. The topsheet fabrics may be composed of a substantially hydrophobic material, and the hydrophobic material may optionally be treated with a surfactant or otherwise processed to impart a desired level of wettability and hydrophilicity.

"Ultrasonic welding" refers to a technology which joins two materials by melting them with heat generated from ultrasonic oscillation and then laminating them together, such that the molten materials flow and fill the gap between the two unaffected portions of the two materials, respectively. Upon cooling and shaping, the two materials are joined together.

By the term "wrapper" as used herein, is meant a packaging component that enclose the absorbent article prior to use. Terms such as "pouch", "wrapping sheet", "packaging sheet" or "wrapping construction" are equivalent to "wrapper".

The term "longitudinal", "transversal" and "vertical" as used herein are with respect to the sanitary napkin in an assembled state or in a disassembled or partially assembled state and in a folded, partially unfolded or unfolded configuration. In other words, the sanitary napkin defines the longitudinal, transversal and vertical directions L,T,V. The term "in the direction" as used herein means along that direction, e.g. in the longitudinal direction means along the longitudinal direction. The terms "top", "bottom", "upper" and "lower" are all in reference to said vertical direction. The term "longitudinally extending" in reference to an element, e.g. a patch of adhesive, as used herein means this element is elongated in the longitudinal direction, meaning that this element is extending more in the longitudinal direction than in the transversal or vertical directions. Similarly, the term "transversally extending" in reference to an element, e.g. a patch of adhesive, as used herein means this element is elongated in the transversal direction, meaning that this element is extending more in the transversal direction than in the longitudinal or vertical directions. Similarly, the term "longitudinally and transversally extending" in reference to an element, e.g. a patch of adhesive, as used herein means this element is extending more in the longitudinal and transversal directions than in the vertical direction. As used herein "longitudinally elongated" means elongated in the longitudinal direction.

All length, width, and height or thickness can be measured with a caliper preferably a micrometer caliper gauge by taking the largest distance separating two points along one direction as defined herein for a given element, e.g. the sanitary napkin in a folded state, the wrapper, or in other words, measuring the distance between two edges of said element along one direction.

The term "length", "width" and "thickness or height" as used herein are respectively in reference to the longitudinal, transversal and vertical direction as defined herein.

The term "bioplastic" used herein refers to any polymeric material, or plastic material, that is biobased, biodegradable/compostable or both. In other words, the term "bioplastic" refers to materials that are either bio-sourced plastics, i.e. derived from biomass, or biodegradable plastics, including compostable plastics. The biodegradable and/or compostable plastics include the plastics derived from fossil resources petrochemical reactions. Some bioplastics have both features bio-sourced and biodegradable.

The term "biodegradable" as used herein refers to a material that when disposed of after use will physically and biologically decompose using known degradation procedures including aerobic, anaerobic, and microbial digestion processes within a specified timeframe, leaving no toxic substances or residue.

The term "compostable" as used herein refers to a material that has the ability to biodegrade under industrial composting conditions including aerobic, anaerobic, and microbial digestion processes within a specified timeframe, leaving no toxic substances or residue.

Embodiments according to the disclosure will now be described. It is understood that technical features described in one or more embodiments maybe combined with one or more other embodiments without departing from the intention of the disclosure and without generalization therefrom.

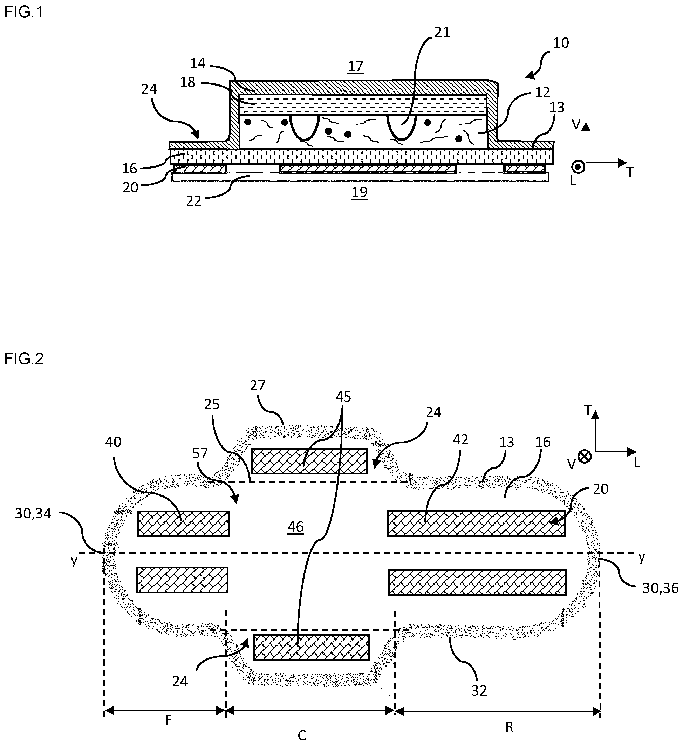

As illustrated in FIG.1, an absorbent article 10 according to the present disclosure selected from and not limited to diapers, training pants, sanitary napkins, sanitary pads, sanitary towels or light incontinence pads comprises an absorbent core 12 arranged between a liquid pervious topsheet 14 and a liquid impermeable backsheet 16. An optional acquisition distribution layer 18, or surge layer, also called ADL 18, can be positioned between said topsheet 14 and said absorbent core 12. The absorbent core 12 comprises absorbent material such as cellulosic fluff pulp with or without superabsorbent polymers. The backsheet 16 is bonded or joined to the topsheet 14 with the absorbent core 12, and optionally the ADL 18, being positioned and enclosed between the topsheet 14 and the backsheet 16. As illustrated in FIG.1, the topsheet 14 and the backsheet 16 are associated with one another in a bonding region 13. In an embodiment, the topsheet 14, backsheet 16 and the ADL 18 are associated altogether in a bonding region 13. The topsheet 14 and the backsheet 16 are associated or joined, directly or indirectly, at the periphery of the absorbent article 10, e.g. a sanitary napkin 10 as illustrated in FIG.2, in said bonding region 13 by attachment means that will be described hereafter. In other words, the bonding region 13 defines the outline, in other words the contour or the perimeter, of said sanitary napkin 10, or the bonding region 13 is at the periphery of the absorbent article 10. The bonding region 13 means the areas where the topsheet and backsheet are bonded to one another directly or indirectly, the bonding region 13 can be a continuous region or discontinuous regions or the bonding region 13 can comprise continuous and discontinuous regions. Simply put, the term "bonding region" means the portion(s) of the absorbent article 10 where the topsheet 14 and backsheet 16 are at least partially associated. The topsheet 14 is arranged on the body-facing side 17 whereas the backsheet 16 is arranged on the garment-facing side 19 of the absorbent article 10. Optionally, the absorbent core 12 and/or the optional ADL 18, can comprise at least one channel 21 or at least one embossment 21 comprising no or less absorbent material. As illustrated in FIG.1, the absorbent core 12 comprises here two channels 21 or two embossments 21. The topsheet 14 and ADL 18 can be embossed as well.

The topsheet 14 consists essentially of natural fibers, or material that is nature-sourced, selected from and not limited to cotton, viscose, hemp, cellulosic fibers, bamboo or a combination thereof. The topsheet can comprise additional materials pigments, softeners, antioxidants, skin care additives such as Aloe Vera, antimicrobial agents such as zinc oxide, odorizing agents, stabilizers, surfactants, waxes, flow promoters or other materials added to enhance processability of the composition provided that these additional materials are also sustainable materials and not plastic material. The topsheet 14 can optionally undergo a hydrophobic treatment on its garment facing side to lower any phenomena of rewet. Natural fibrous materials such as cotton are more sustainable however they are less malleable than plastic materials and are not heat-sensitive. In particular natural fibers do not melt at lower temperatures (< 200°C, for example cotton burns at 400°C). The topsheet 14 being intended to be in direct contact, or in close proximity, with the skin, having such material with natural fibers is also preferable for dermatologic reasons.

The backsheet 16 is intended to act as a liquid barrier retaining the liquid within the absorbent core 12 and is arranged furthest from the skin, *i.e.* in proximity of the garment. The backsheet 16 may be a unitary layer of material or may be a composite layer composed of multiple components assembled side-by-side or laminated. The backsheet 16 may be the same or different in different parts of the absorbent article 10. At least in the area of the absorbent core 12, the backsheet 16 comprises a liquid impervious material in the form of a thin plastic film such as a polyethylene or polypropylene film, a nonwoven material coated with a liquid impervious material, a hydrophobic nonwoven material, which resists liquid penetration, or a laminate of a plastic film and a nonwoven material or a plastic derived from fossil resources petrochemical reactions. The backsheet material may be breathable so as to allow vapour to escape from the absorbent material, while still preventing liquids from passing there through. Examples of breathable backsheet materials are porous polymeric films, nonwoven laminates of spunbond and meltblown layers and laminates of porous polymeric films and nonwoven materials. The backsheet 16 can also comprise, for example consists essentially of, bioplastic meaning a plastic material that is bio-based or bio-sourced, biodegradable/compostable or both. In other words, the backsheet 16 can comprise a material that is either bio-sourced plastics, i.e. derived from biomass, and/or that is biodegradable plastics, including compostable plastics. Such material can be selected from, and not limited to, the followings: polyhydroxy butyrate (PHB), thermoplastic starch (TPS), polyvinyl alcohol (PVOH), polylactic acid (PLA), polyethylene oxide (PEO), Arboblend^{®}, polyhydroxylakanoate (PHA), polycaprolactone (PCL) and bio-polyethylene (bio-PE), namely low-density polyethylene (LDPE). Such material is malleable but melts at lower temperatures (< 200°C, for example polyethylene melts around 130°C).

The association, or the bonding, of a layer of material that is rigid and melting at high temperature (greater than 200°C) to a layer of material that is malleable and melting at lower temperature (below 200°C) is an issue and using common attachment means such as applying pressure and heating (traditional crimping) does not function well, meaning there a risk that the topsheet and backsheet dissociate from one another during use of the absorbent article 10. Additionally, the bonding of the topsheet 14 to the backsheet 16 has to be done at the periphery of the absorbent article 10, in the bonding region 13, limiting the space available for bonding one sheet to the other.

The topsheet 14 comprises natural fibers and preferably has a basis weight comprised between 10 and 50 gsm, preferably between 25 and 40 gsm. The topsheet can have a thickness comprised between 0.40 and 0.80 mm. The topsheet can have an apparent density comprised between 12.5 and 125 kg.m⁻³, preferably between 31.25 and 100 kg. m⁻³. The topsheet 14 can optionally be apertured and/or embossed in the area within the bonding region 13. The backsheet 16 comprises liquid impermeable material, for example the backsheet 16 corresponds to a bio-polyethylene (bio-PE) SMS (Spunbond-Meltblown-Spunbond) laminate. The backsheet 16 preferably having a basis weight comprised between 8 and 25 gsm, preferably between 10 and 20 gsm. The backsheet can have thickness between 20 and 80 µm.

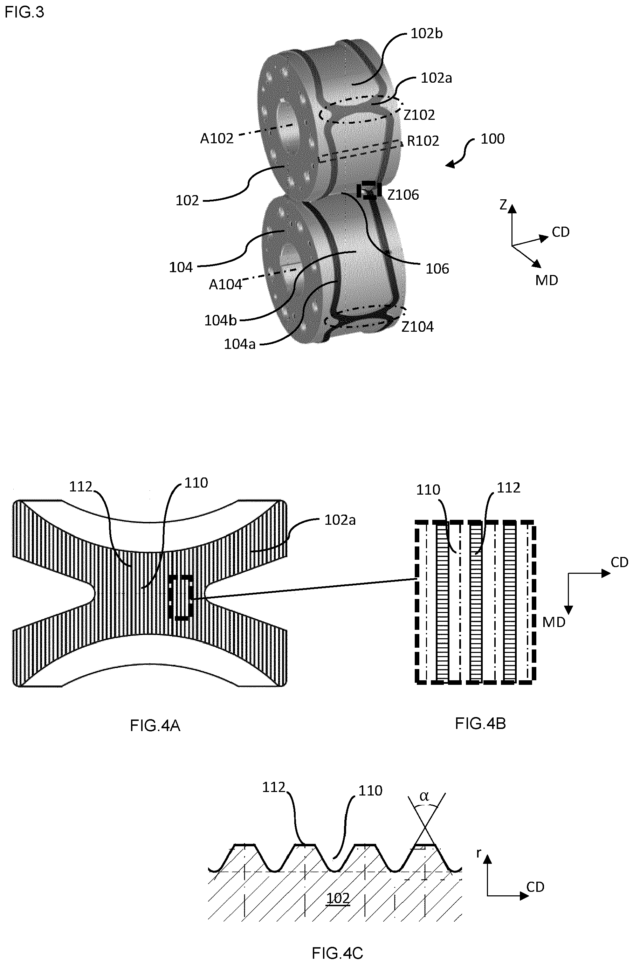

To ensure a proper joining between the topsheet 14 and the backsheet 16, the bonding is preferably carried out using an apparatus 100 as illustrated in FIG.3. The apparatus 100 for bonding a cotton-based, or any other natural material-based topsheet and a plastic material-based backsheet comprises a first bonding roller 102 and a second bonding roller 104 forming together a nip 106 through which the absorbent articles 10, e.g. topsheet 14, absorbent core 12, ADL 18 and backsheet 16, pass through. Both bonding rollers 102,104 are cylindrical rollers each rotating around respective rotation axis A102,A104 (FIG.3). The first and second bonding rollers 102,104 both comprise positive and negative imprints that will be describes hereunder in order to ensure a proper bonding. The apparatus 100 comprises, preferably consists of, two corresponding cylindrical bonding roller 102,104, from which the relevant bonding diameters is simultaneously equal and greater than the drive diameters, at any given time. More precisely, the diameter of the relevant bonding roller is equal and greater than the drive diameter due to the positive and negative imprints ensuring the bonding in the bonding region 13 and equal due to the flat surface where the absorbent core is arranged. As explained previously, natural fibers are not heat sensitive hence conventional methods such as heat crimping as not well adapted. The bonding apparatus should promote or favour mechanical bonding to ensure a proper mechanical connection, meaning a proper entanglement of the fibers of the topsheet 14 with the fibers of the backsheet 16 and eventually ADL 18. It is also possible according to an optional embodiment to incorporate heating elements in the apparatus 100 to promote the malleability of the backsheet.

The first bonding roller 102 comprises on its circumferential outer surface a first bonding surface 102a, or a first bonding pattern 102a, and a first flat surface 102b. The first bonding surface 102a is arranged to define the bonding region 13 of the absorbent article, meaning the first bonding surface 102a is arranged to define the periphery or contour or perimeter, of an absorbent article 10 and the first flat surface 102b is arranged at least within the area defined by the first bonding surface 102a as illustrated in FIG.3. The first bonding roller 102 comprises several first bonding patterns 102a so as to bond several absorbent articles 10 in one revolution, meaning in a rotation of 360°. In other words the first bonding surface 102a is arranged with repeated motifs, three as illustrated in FIG.3, so that when the first bonding roller 102 undergoes a full revolution, three absorbent articles 10, specifically three topsheets 14 and backsheets 16, will be bonded together along the periphery of their respective absorbent article 10. Another way to say this is that the first bonding surface 102a comprises three identical and repeated first bonding patterns. Naturally, the disclosure is not limited to three motifs, it can be one, two, four, five, six, seven, eight, nine, ten or more repeated bonding patterns. The same features are present in the second bonding roller 104 comprising a second bonding surface 104a with repeated second bonding patterns 104a or motifs, and a second flat surface 104b.

The first bonding pattern 102a comprises a plurality of grooves 110 separated by ridges 112, or crests 112, as illustrated in FIG.4A and 4B which both represent a close-up of the first bonding roller 102, namely in the area Z102 (FIG.3), when seen in the radial direction, meaning when seeing in direction of the center of the cylindrical first bonding roller 102. The bonding pattern 102a comprises an assembly of grooves 110 extending in parallel directions. As illustrated in FIG.4A and 4B, the grooves 110 and ridges 112 all extend, meaning are elongated, in one direction, in other words the grooves 110 and ridges 112 are parallel to one another, here in the machine direction MD, meaning in the same direction as the webs of tospheet and backsheet are being conveyed. The grooves 110 can all extend in another direction such as in the cross-direction CD or in diagonal relative to the machine direction, the grooves 110 being preferably all extending, or being elongated, in parallel directions. The grooves 110 are defined by ridges 112 or crests 112, also extending parallel to one another, in the same directions as the grooves 110 that they define or vice versa. The grooves 110 correspond to the negative imprints whereas the ridges 112 correspond to the positive imprints.

The second bonding roller 104 comprises on its circumferential outer surface a second bonding pattern 104a and a flat surface 104b. The second bonding pattern 104a also defines the periphery, meaning the contour, of an absorbent article 10. The second bonding roller 104 comprises several second bonding patterns 104a so as to bond several absorbent articles 10 in one full rotation (i.e. rotation of 360°) as described hereabove. In other words the second bonding pattern 104a is arranged in repeated motifs, three as illustrated in FIG.3, so that the when the second bonding roller 102 makes a full revolution, three absorbent articles, specifically three topsheets 14 and backsheets 16, will be bonded together along the periphery of their respective absorbent article 10. Naturally, the disclosure is not limited to three motifs, it can be one, two, four, five, six, seven, eight, nine, ten or more repeated bonding patterns.

The first and second bonding surfaces 102a,104a are in facing relationship or in mirror relationship in order to define together a proper periphery of the absorbent article 10 as illustrated in FIG.2. In other words, the first and second bonding surfaces 102a,104a are arranged in a way that the first and second bonding surfaces 102a,104a bonding the longitudinal sides 32 of the absorbent article are synchronized same as the first and second bonding surfaces 102a, 104a bonding the transversal sides 30 of the absorbent article 10.

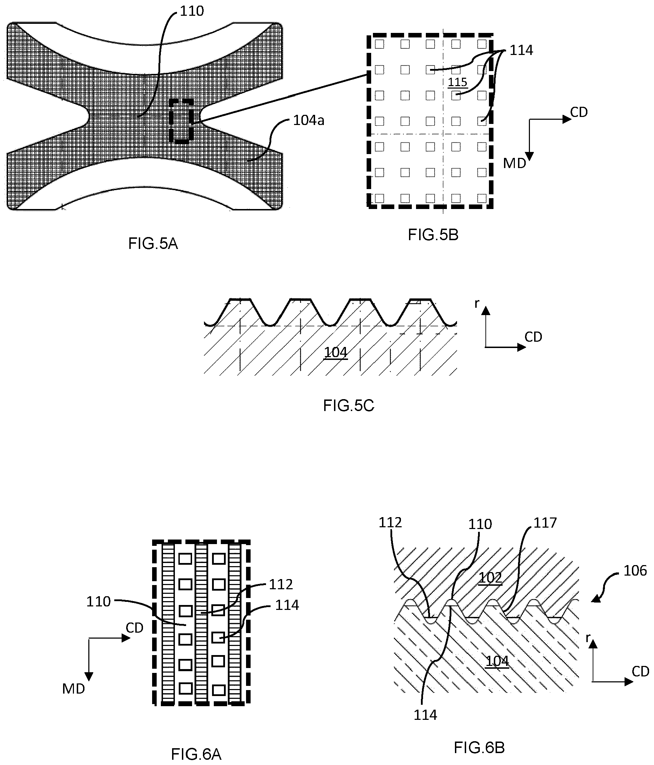

The second bonding pattern 104a comprises a pattern of isolated protrusions 114 as illustrated in FIG.5A and 5B. The second bonding pattern 104a comprises an assembly of protrusions 114 aligned in different parallel lines. As illustrated in FIG.5A, the protrusions are aligned in different parallel lines extending for example in the machine direction MD, meaning in the same direction as the webs of tospheet and backsheet are being conveyed. The protrusions 114 can all be aligned in another direction such as in the cross-direction CD or in diagonal relative to the machine direction.

The term "bonding pattern" and "bonding surface" are interchangeable terms meaning a surface with a pattern enabling to deform or stamp said pattern, i.e. the bonding pattern, on a object such as one or more sheets so that it can stands out, or be raised, in relief. By arranging two bonding or embossing surfaces or two bonding surfaces in facing relationship, the sheets passing between the two surfaces, in the nip 106, are deformed and the fibers of said sheets are deformed and entangled thereby forming a bonding point or bonding site.

The protrusions 114 are arranged to fit within said grooves 110 as illustrated in FIG.6. More specifically, the protrusions 114 are aligned in the same direction as the direction of extension of the grooves 110. Within the nip 106, meaning when the points on the circumference of the two bonding rollers 102,104 are in the closest proximity *i.e.* the position where the distance between the points on the circumference of the two bonding rollers is least, the protrusions 114 are at least partially nested within the grooves 110 as illustrated in FIG.6B.

Such arrangement enables to ensure a good enough deformation of the material and force a mechanical entanglement of the natural fibers, meaning the fibers of the topsheet 12 and the plastic fibers, meaning the fibers of the backsheet 16. Additionally, by having continuous grooves 110 on a bonding roller, e.g. the first bonding roller 102, there is not need to add a dedicated synchronism mechanism between the two roller 102,104 thereby additionally saving on cost. Of course there is still a need to ensure that end-bonding regions e.g. Z102, Z104 are in facing relationship in the nip 106, but a visual check and ensuring that the rollers 102,104 rotate at the same speed is sufficient.

The first bonding roller 102 or the second bonding roller 104 or a combination of both can be heated to ease the deformation of the material, namely the backsheet 16.

The first bonding roller 102, for example the cylindrical roller arranged on top as illustrated in FIG.3, comprises a plurality of circumferential crests or ridges 112 separated by circumferential grooves or valleys 110. While the crests or ridges 110 may be of varying cross-sectional shape, the crests 110 are preferably of the shape illustrated in FIG.4C, that is, generally truncated right rectangular pyramids, or frusto-pyramidal, projecting from the circumferential outer surface of the first bonding roller 102 in the radial direction r. The separating grooves or valleys 110 on the first bonding roller 102 with two grooves 110 being arranged on each side of a crest 112 with respect to the cross direction are U or V-shaped with an angle from the vertical or radial direction. According to an embodiment, both sides of a ridge 112 of first bonding roller 102 form together an angle α (FIG.4C), comprised between 20° and 90°, preferably between 40° and 80°, preferably 60°.

The second bonding roller 104, for example the cylindrical roller arranged on bottom as illustrated in FIG.3, comprises a plurality of protrusions 114 isolated from one another by a gap 115. While the protrusions 114 may be of varying cross-sectional shape, the protrusions 114 are preferably of the shape illustrated in FIG.5C that is, generally truncated right rectangular pyramids, or frusto-pyramidal, projecting from the circumferential outer surface of the second bonding roller 104 in the radial direction r. The protrusions 114 are in the shape of frusto-pyramids, or truncated pyramids, projecting from the circumferential outer surface of the second bonding roller 104. The different protrusions 114, or pyramids, are separated from one another in the machine and the cross directions CD,MD by a gap 115 from the next one. As illustrated in FIG.5B, the protrusions 114 are aligned in the cross-direction CD and/or in the machine direction MD, such alignment enables the protrusions 114 to be nested withing a groove 110 as illustrated in FIG.6A.

The protrusions 114 are not limited to this shape alone. For example the protrusions can correspond to truncated elliptical cones that decrease in surface from the base, corresponding to the flat surface of the second bonding roller 104, up to its apex. Whatever the shape, preferably each protrusion 114 is identical to the other and presents a squared, rectangular or oblong surface at its apex. Starting from the apex, each protrusion 114 widens in direction of the base. According to an embodiment, each protrusion 114 presents at its apex an oblong surface with two opposite sides being larger than the two other opposite sides, in other words, the apex surface of each protrusion extends, or is elongated, sensibly in one direction.

The protrusions 114 can be arranged in a grid configuration where the columns and rows are all aligned respectively or the protrusions 114 can be arranged in a staggered configuration, where the rows are aligned two by two with two adjacent rows being shifted by a pitch.

The protrusions 114 are dimensioned so that the apex having a CD dimension in the range of about 0.25 mm to about 1 mm, preferably about 0.5 mm, a MD dimension of about 0.25 mm to about 1 mm, preferably about 0.5 mm. As illustrated in FIG.5B, the apex is preferably squared-shape, meaning when seen from above, or in the radial direction, the apex defines a square preferably with a surface area of 0.25 mm². The axial distance between the base, meaning the flat surface of the circumferential outer surface defined by the gap 115, and the apex is in the range of about 0.25 mm to about 1 mm, preferably about 0.67 mm, and the sidewalls diverging from the apex at an angle to one another in the range of about 20° to about 90°, preferably about 60°. The center of each apex, or the center of each protrusions 112, are separated by a distance of about 1 mm to about 2 mm, preferably 1.5 mm.

Similarly, the center of each groove 110 is separated from the next center of an adjacent groove by a distance of about 1 mm to about 2 mm, preferably 1.5 mm in order to fit the protrusions 114. The axial distance between the groove and the apex of the crest 112 is in the range of about 0.25 mm to about 1 mm, preferably about 0.67 mm, and the sidewalls diverging from the apex at an angle to one another in the range of about 20° to about 90°, preferably about 60°.

With such dimensions, the protrusions 114 and grooves 110 and crests or ridges 112 are well nested together thereby ensuring a good entanglement of the natural fibers with the plastic fibers.

With such bonding apparatus 100, the bonding of the topsheet 14 with the backsheet 16 and optionally the ADL 18 is strong enough to withstand a force up to 1.5 N or even 2 N, meaning when exercising a force of 2 N or 1.5 N or less, the two or three layers stay bonded or laminated.

The mechanical bonding comprises active contact edges 117 (FIG.6B) which is the portions of the rollers 102 and 104 located between the two apex of the first and second bonding roller 102,104 in the nip region 106, meaning the portion of the two rollers 102,104 between the apexes of the ridges 112 and protrusions 114, when said rollers 102,104 are in the closest proximity. Each active contact edge 117 has a absolute length or distance comprised between about 0.25 mm and about 1 mm preferably about 0.5 mm. For example, the active contact edge 117 has a same dimensions as the protrusions, i.e. if the protrusions has a length of 0.5 mm relative to the cross direction and/or machine direction, than the active contact edge 117 has an absolute length of 0.5 mm for example relative to the radial and cross direction as illustrated in FIG.6B.

Preferably the bonding roller impart a total force, i.e. a total bonding force, comprised between 10 and 25 kN, preferably between 15 and 20 kN. Such force is high enough to promote the entanglement of the natural fibers of the topsheet 14 with the fibers of the backsheet 16 and eventually with the fibers of the ADL 18 without damaging the sheets, meaning such force enables to bond the two, or three, sheets i.e. topsheet, backsheet, ADL, without inducing tearing or shearing.

Given the shape of the bonding region 13 and its area, the total bonding force per area varies depending on the region of the bonding regions 13, i.e. the bonding regions at the longitudinal sides 30 or the bonding regions at the transversal sides 32. For example, as exemplified in FIG.3, the force per bonding surface area is greater at the zone Z102 than at the zone Z106, given that the bonding area Z106 is greater than the bonding area in the zone Z102. For example, at the radial position of the bonding roller illustrated Z102, the distance, or width here, of the bonding region 13 is between 2 and 6 mm with respect to the cross direction, whereas at radial position illustrated Z106, the distance or width of the bonding region 13 is between 40 to 80 mm with respect to the cross direction. These widths represents a different contact surface combining the different discrete, or intermittent, active contact edges 117 aligned on these said widths as illustrated in FIG.6B. Given that the force imparted is the same, between 10 to 25 kN, hence the force per contact surface is different depending on the zone of the bonding region 13. For example, considering at a radial position, a rectangle R102 (FIG.3) extending in the cross and machine directions, said rectangle R102 being 1 mm wide in the machine direction and as long as the first bonding roller 102 in the cross direction, in that case, the bonding region extends on 8 mm (4+4 mm) in the cross direction and on 1 mm in the machine direction. There would then be 0.5 mm² of contact surface per active contact edges 117 with 10 actives contact edges 117 that would amount to 5 mm² of contact surface in that rectangle R102. Another way to phrase this is by considering that there is 5 mm of linear contact for 8 mm of bonding area. Hence the force per linear contact is comprised between 2 and 5 kN.mm⁻¹ (10 to 25 kN (force) divided by 5mm (linear contact)). The same logic applies to a rectangle or a line in the region Z102 or Z104 in FIG.3 with 40 mm of linear contact for 60 mm of bonding region, hence a force per linear contact comprised between 0.25 and 0.625 kN.mm⁻¹. To summarize the force per linear contact is comprised between 0.1 and 10 kN.mm⁻¹, preferably between 0.25 and 5 kN.mm⁻¹ as such force enables a proper bonding with inducing tear and shearing.

As seen previously, the bonding region 13 comprises discrete or intermittent bonding sites with the protrusions 114 and ridges 112 being isolated or spaced apart from one another. The bonding rollers 102,104 comprise in the nip 106 region a bonding pattern having a bonding density of between 60 and 95%, preferably comprised between 70 and 90%, of the area circumscribed by the bonding pattern including the non-bonded area, meaning the gaps 115, between bonding sites. The bonding density is defined herein as the bonded area per centimeter, in the machine direction, of the pattern surface. For the purpose of establishing a bonding density it is necessary to consider regions which fully encompass the bonding pattern, i.e. excluding regions which have no bonding pattern such as the flat surfaces 102b,104b. The bonded area defined herein may refer to the bonded area of the topsheet to the backsheet, or the bonded area may refer to the corresponding area of the pattern surface, comprising a plurality of protrusions or pins. The term "bonded area" means the area of a bonding pattern that is occupied by bonding elements, meaning the ridges 112 and protrusions 114. The term "bonding density" means the area occupied by bonding elements meaning the ridges 112 and protrusions 114 in relation to the entire area circumscribed by the bonding pattern including the non-bonded areas or gap 115 between bonding elements. It is preferable to have a high bonding density to ensure a proper entanglement of the fibers of the topsheet 14 and backsheet 16. In other words, the bonding region 13 comprises discrete attachment sites, said discrete attachment sites or points are arranged with a bonding density comprised between 60 and 95%.

Other means for bonding natural-material based fibers with plastic fibers can be used. For example, document US5269860 relates to a method of ultrasonically bonding a thermoplastic sheet such as polypropylene or polyester to a non-thermoplastic fibrous article such as a cotton fabric using ultrasonic energy between about 10 and about 50 KHz, preferably between about 15 KHz and about 20 KHz or hydroentanglement also can be used. Although, whilst these methods do not require any adhesive, they can be complicated to implement for such a precise bonding at the periphery of the article and they require high levels of energy.

Hence the present disclosure also pertains to an absorbent article 10 where the topsheet 14 and the backsheet 16 are attached, directly or indirectly via the ADL 18, by ultrasonic bonding to one another in a bonding region 13, said bonding region 13 being positioned along the periphery of the absorbent article 10, said bonding region 13 being free of adhesive. In other words, the apparatus comprises the second bonding roller 104 as described herein and an ultrasonic welding device, said ultrasonic welding device comprising a vibration generator, for example and not limited to, a magnetostrictive or piezoelectric transducer, said generator being attached to a tapering rod or probe usually made out of metal, such as and not limited to titanium, aluminum or steel, i.e. the bonding sonotrode. The bonding sonotrode is submitted to the vibrations generated by the generator and said bonding sonotrode then applies this vibrational energy to the combined first and second sheet materials. The vibrational energy causes the partial melting of the backsheet 16 and the pressure exerted by the embossment roller 104 ensures a proper entanglement and thus bond between the topsheet 14 and the backsheet 16 without any adhesive. The sonotrode emitting ultrasonic vibrations at a frequency between 10 kHz and 70 kHz, preferably between 15 kHz and 40 kHz. Such frequency is high enough to promote the entanglement of the natural fibers of the topsheet 14 with the fibers of the backsheet 16 and eventually with the fibers of the ADL 18 without damaging the sheets, meaning such force enables to bond the two, or three, sheets i.e. topsheet, backsheet, ADL, without inducing tearing or shearing.

In an embodiment where the absorbent article 10 corresponds to a sanitary napkin 10, the sanitary napkin 10 comprises on the garment-facing side 19 of the backsheet 16 at least one layer of adhesive 20 so that the sanitary napkin 10 can adhere onto a garment or undergarment, said at least one layer of adhesive ensuring that the absorbent article 10 stays fastened and secured on the underwear. In order to maintain the structural integrity of the adhesive before being used, the sanitary napkin 10 comprises a release paper 22 corresponding to a peelable layer destined to protect the at least one layer of adhesive. The release paper is preferably a siliconized paper acting as a release liner.

A sanitary napkin 10 is illustrated in FIG.2 in an unfolded configuration, e.g. laid flat on a surface. The sanitary napkin 10 extends in the longitudinal, transversal and vertical directions L, T, V as illustrated in FIG.1 and 2, thereby defining a length in the longitudinal direction L, a width W in the transversal direction T and a thickness or height in the vertical direction V. In an unfolded configuration as shown in FIG.2, the length of the sanitary napkin 10 is greater than the width.

The sanitary napkin 10 comprises a topsheet 14, a backsheet 16, an absorbent core 12 arranged between said topsheet 14 and backsheet 16, and two foldable wings 24 or flaps 24 arranged at opposite sides of the sanitary napkin 10 with respect to the transversal direction T. As illustrated in FIG.2, the sanitary napkin 10 extends in longitudinal, transversal and vertical directions L,T,V, the sanitary napkin has a longitudinal centerline, or central axis, y-y, a pair of transversal sides 30, meaning a pair of sides arranged at each longitudinal ends or longitudinal extremities and extending in the transversal direction, of the sanitary napkin 10, and a pair of longitudinal sides 32 extending therebetween, meaning a pair of sides arranged along each transversal ends of the sanitary napkin 10 and connecting the transversal sides 30 to one another and extending in the longitudinal direction. Similarly, the pair of transversal sides 30 connects the two longitudinal sides 32 to one another. In other words, the sanitary napkin 10 comprises two ends, or extremities, at least partially extending in the transversal direction T, *i.e.* the transversal sides 30, and two ends at least partially extending in the longitudinal direction L, *i.e.* the longitudinal sides 32. As illustrated in FIG.2, the transversal sides 30 are arcuate whereas the longitudinal sides 32 are linear. The sanitary napkin 10 has a front edge 34 and a rear edge 36 positioned on opposite apexes of said transversal sides 30, meaning at each longitudinal extremities of the sanitary napkin 10. The sanitary napkin 10 is divided with respect to the longitudinal direction L, into three sections or portions, a front portion F, a rear portion R and a central portion C arranged between said front and rear portions F,R. The sanitary napkin 10 also comprises wings 24, or flaps 24, arranged at each transversal end of the sanitary napkin 10, the wings 24 extend transversely beyond the longitudinal sides 32 of the sanitary napkin 10. In other words, the flaps 24 are transversally, or laterally, outboard of the longitudinal centerline y-y and central portion C of the sanitary napkin 10. In other terms, each flap 24 comprises a proximal end 25 and a distal end 27 with respect to the longitudinal centerline y-y and with respect to the transversal direction T. Said proximal end 25 is connected to the sanitary napkin 10, *i.e.* connecting the sanitary napkin 10 and the wing 24 and said distal end 27 is the point furthest from the longitudinal centerline y-y. As used herein the term "central portion" C refers to the portion of the sanitary napkin 10 arranged in between the front and rear portions F, R and in between the proximal ends 25 of the flaps 24, meaning intermediate, particularly laterally intermediate of the two wings 24. In other words, the central portion C is defined by the length of the wings 24 taken in the point in which they extend from the longitudinal sides 32 of the sanitary napkin 10 in a direction parallel to the longitudinal axis y-y. In other words, the central portion C is delimited longitudinally by the front and rear portions F, R and transversally by the proximal ends 25 of the flaps 24. The wings 24 may be comprised of an integral and contiguous extension of the topsheet 14, the backsheet 16, or a laminate of both. Alternatively, the wings 24 may be made of separate and independent pieces of material joined to the longitudinal sides 32 of the sanitary napkin 10. Each wing 24 has one face generally coextensive of the topsheet 14 and a mutually opposed face generally coextensive of the backsheet 16.

As illustrated in FIG.2, the sanitary napkin 10 comprises two regions, a main body 57 and the wings 24. The main body 57 is the region, or portion of the absorbent article 10 comprising the absorbent core 12 and the optional ADL 18 and is oblong or oblong-shaped, meaning comprising a shape, e.g. a rectangular, that is longer than it is wide and whose corners are rounded. The wings 24 correspond to rectangular or trapezoidal extensions outboard of the main body 57, meaning outboard of the oblong shape, with respect to the transversal direction T. The wings 24 are inboard of the main body 57 with respect to the longitudinal direction L and are arranged in the central portion C of the sanitary napkin 10 with respect to the longitudinal direction L. The main body 57 can be rectangular. The wings 24, only comprising a laminate of topsheet 14 and backsheet 16, are flexible and foldable, meaning the wings 24 can be easily pivoted or rotated at least around a longitudinal axis. The topsheet 14 and the backsheet 16 are associated with one another along the periphery of the sanitary napkin 10 at the main body 57 region and at the wings 24 in bonding regions 13. The pair of longitudinal sides 32 are in reference to the main body 57.

The wings 24 preferably comprises means for attaching one face of the wing 24 to the wearer's undergarment or to the other wing 24. The wings 24 comprises at least one layer of adhesive 20. The attachment means may be pressure sensitive adhesive. If pressure sensitive adhesive is elected, it should be disposed on the face of the wing generally coextensive of the backsheet 16 so that when the wing 24 are wrapped around the crotch portion of the wearer's undergarment, the layer of adhesive will contact the outside of the wearer's undergarment. The sanitary napkin 10 for example comprises a generally rectangular patch of adhesive on each wing 24.

The sanitary napkin 10 comprises at least one layer of adhesive 20 to fasten the sanitary napkin 10 to an undergarment. In order to protect the layer of adhesive 20 prior to its use, the sanitary napkin 10 comprises either a release paper 22 as illustrated in FIG.1 and/or a pouch, to cover directly or indirectly said at least one layer of adhesive 20 each release paper 22 and/or pouch preferably comprising a layer or coating of peeling agent. Said at least one layer of adhesive 20 is arranged on one side of the backsheet 16, namely on the garment-facing side 19 of the backsheet 16, the backsheet 16 being arranged between the layer of adhesive 20 and absorbent core 12 as illustrated in FIG.1. In other words, the at least one layer of adhesive 20 is arranged between the backsheet 16 and the release paper 22 and/or pouch. As illustrated in FIG.2, the at least one layer of adhesive 20 is arranged on the backsheet 16 in a discontinuous pattern of adhesive wherein said pattern of adhesive comprises a first area of adhesive 40 arranged in the front portion F, a second area of adhesive 42 arranged in the rear portion R and a third area of adhesive 45 being arranged on each wing 24. The third area of adhesive 45 is arranged between the proximal and distal ends 25,27 of each wing 24. The pattern of adhesive comprises an adhesive-free area 46, or a gap free of adhesive, between the first area of adhesive 40 and the second area of adhesive 42 where no adhesive is present, said adhesive-free area 46 longitudinally extending on a length that is greater than or equal to the length of the third area of adhesive 45 with respect to the longitudinal direction L. In other words, the central portion C substantially does not comprise any adhesive, or there is no adhesive in the central portion C as defined hereabove. As illustrated in the drawings, the adhesive-free area 46 is in relation to the main body 57, meaning the portion of the absorbent article 10 comprising the absorbent core 12 and the eventual ADL 18 and is oblong or oblong-shaped. More specifically, the first and second areas of adhesive 40,42 are arranged on the main body 57, i.e. on the backsheet at the main body portion, with an adhesive-free area 46 arranged in between said first and second areas of adhesive 40,42 and the third area of adhesive 45 is arranged on the wings 24 which are outboard of the main body 57 with respect to the transversal direction T. The first and second areas of adhesive 40,42 can extend partially within the central portion as illustrated in FIG.5 and FIG.4A without departing from the invention as long as an adhesive-free area 46 is present between the first and second areas of adhesive 40,42 within the central portion C, meaning within the central portion C of the main body 57 portion of the sanitary napkin 10.

The length of the first area of adhesive 40 can be more or less equal to the length of the third area of adhesive 45 and the length of the first area of adhesive 40 is lesser than the length of the second area of adhesive 42. Hence, the length of the first area of adhesive 40 can be more or less equal to the length of the adhesive-free area 46, "length" as used herein being the distance of the element, i.e. patch of adhesive or adhesive-free area or gap, in the longitudinal direction L.

According to an embodiment, the ratio of the length of the first area of adhesive 40 to the length of the third area of adhesive 45 is comprised between 0.8 and 1.2, preferably between 0.9 and 1.1. The ratio of the length of the first area of adhesive 40 to the length of the second area of adhesive 42 is comprised between 0.4 to 0.9, preferably between 0.5 and 0.7. The ratio of the length of the first area of adhesive 40 to the length of the adhesive-free area 46 is comprised between 0.8 and 1.2, preferably between 0.9 and 1.1. The ratio of the length of the adhesive-free area 46 to the length of the third area of adhesive 45 is comprised between 0.8 and 1.2, preferably between 0.9 and 1.1, more preferably about 1. Again, "length" as used herein being the distance of the element, i.e. patch of adhesive or adhesive-free area or gap, in the longitudinal direction L. Such arrangement enables that the adhesive pattern is continuously attached to the undergarment on one side or the other thereby still ensuring a good fastening of the sanitary napkin 10 onto the undergarment.

With such an adhesive pattern, it is possible to further save on material and use even less adhesive. The combination of having this adhesive pattern and using attachment means as described hereabove is particularly beneficial to save on adhesive and have more sustainable absorbent articles. It is also possible with this pattern of adhesive to fold the wings 24 outward, meaning toward the backsheet 16 since the adhesive normally present in the central portion C cannot hinder the unfolding of the wings 24. As illustrated in FIG.3A, with such configuration, the wings can be folded onto the backsheet without the layer of adhesive 20 present on the backsheet 16, specifically the layer of adhesive 20 arranged on the backsheet 16 in the front and rear portions F,R, adhering to the wings 24. Of course, it is also possible to fold the wings 24 inward toward the topsheet 14 and still save on material. In other words, comprising a gap meaning an area free of adhesive, between the first and second areas of adhesive 40,42, offers the possibility to fold the wings onto the topsheet 14 or onto the backsheet 16, said adhesive-free area 46 longitudinally extending on a length that is greater than or equal to the length of the third area of adhesive 45 with respect to the longitudinal direction L. Preferably, the adhesive-free area46 is longitudinally extending on a length that is more or less equal to the length of the third area of adhesive 45 with respect to the longitudinal direction L, such arrangement enables that the adhesive pattern is continuously attached to the undergarment on one side or the other thereby still ensuring a good fastening of the sanitary napkin 10 onto the undergarment.

According to an embodiment, the third area of adhesive 45 is skewed or shifted with first and second areas of adhesive 40, 42 when all areas of adhesive area projected on the longitudinal centerline y-y. In other words, the first, second and third areas of adhesive 40,42,45 do not overlap when projected on a longitudinal axis.

The present disclosure also pertains to a method suitable for manufacturing an absorbent article 10 as described herein, the method comprising:
- providing a topsheet 14 consisting essentially of natural fibers, such as a cotton sheet,
- providing a backsheet 16 comprising plastic material, such as a bio-ipolyethylene SMS laminate,
- arranging an absorbent core 12 comprising absorbent material in between said topsheet 14 and backsheet 16,
- applying a mechanical bonding with an apparatus described herein, in said bonding region 13 along the periphery of the absorbent article 10 to bond said topsheet 14 to said backsheet 16, said bonding region 13 being free of adhesive.

Alternatively, the present disclosure also pertains to a method suitable for manufacturing an absorbent article 10 as described herein, the method comprising:
- providing a topsheet 14 consisting essentially of natural fibers, such as a cotton sheet,
- providing a backsheet 16 comprising plastic material, such as a PE film,
- arranging an absorbent core 12 comprising absorbent material in between said topsheet 14 and backsheet 16,
- applying an ultrasonic bonding with an apparatus comprising:
   a. an ultrasonic welding device comprising a sonotrode inducing vibrations at a frequency between 10 kHz and 50 kHz, preferably between 15 kHz and 30 kHz.
   b. a second bonding roller 104 having a circumferential outer surface comprising a second flat surface 104b and second bonding surface 104a, said second bonding surface comprising a plurality of protrusions 114,
wherein the second bonding surface 104a is arranged to define the periphery of at least one absorbent article 10 and said second flat surfaces 104b being arranged within the areas defined by the first and second bonding surface 104a respectively, said ultrasonic bonding being applied in said bonding region 13 along the periphery of the absorbent article 10 to bond said topsheet 14 to said backsheet 16, said bonding region 13 being substantially free of adhesive.

## Claims

1. Absorbent article (10) comprising :
- a liquid pervious topsheet (14) consisting essentially of natural fibers,
- a liquid impervious backsheet (16) comprising a plastic material,
- an absorbent core (12) arranged between said topsheet (14) and backsheet (16) comprising absorbent material,
wherein the topsheet (14) and the backsheet (16) are attached via attachment means to one another in a bonding region (13), said bonding region (13) being positioned along the periphery of the absorbent article (10),
**characterized in that** said bonding region (13) is substantially free of adhesive.

2. Absorbent article (10) according to claim 1, wherein said bonding region (13) comprises discrete attachment points.

3. Absorbent article (10) according to claim 2, wherein said discrete attachment sites are arranged with a bonding density comprised between 60 and 95%.

4. Absorbent article (10) according to any of the claim 1 to 3, wherein the absorbent article (10) further comprises an acquisition distribution layer (18) arranged between the topsheet (14) and the absorbent core (12), said acquisition distribution layer (18) being arranged between the topsheet (14) and the backsheet (16) in the bonding region (13), the topsheet (14), acquisition distribution layer (18) and the backsheet (16) being attached altogether via said attachment means in the bonding region (13).

5. Absorbent article (10) according to any of the claims 1 to 4, wherein the absorbent article (10) is a sanitary napkin (10).

6. Absorbent article (10) according to claim 5 , wherein said sanitary napkin (10) further comprises two wings (24), said sanitary napkin (10) extending in longitudinal, transversal and vertical directions (L,T,V), the sanitary napkin (1) being divided with respect to the longitudinal direction, into a front portion (F), a rear portion (R) and a central portion (C) arranged between said front and rear portions, the central portion (C) being defined by the length of the wings (24) taken in the point in which they extend from the longitudinal sides (32) of said sanitary napkin (10) in a direction parallel to the longitudinal axis (y-y),
wherein a layer of adhesive (20) is arranged on the side of the backsheet (16) opposite to the absorbent core (12),
**characterized in that** said layer of adhesive (20) is arranged on the backsheet (16) in a discontinuous pattern, said pattern of adhesive comprising:
- a first area of adhesive (40) arranged in the front portion (F),
- a second area of adhesive (42) arranged in the rear portion (R);
- a third area of adhesive (45) being arranged on the wings (24);
wherein said pattern of adhesive comprises an adhesive-free area (46) between the first area of adhesive (40) and the second area of adhesive (42), said adhesive-free area (46) longitudinally extending on a length that is greater than or equal to the length of the third area of adhesive (45) with respect to the longitudinal direction (L).

7. Apparatus (100) for bonding a liquid pervious topsheet (14) consisting essentially of natural fibers to a liquid impervious backsheet (16) comprising a plastic material, said apparatus (100) comprising
a. a first bonding roller (102) having a circumferential outer surface comprising a first flat surface (102b) and a first bonding surface (102a), said first bonding surface (102a) comprising a plurality of grooves (110) and ridges (112) extending in the machine direction (MD) and/or cross direction (CD);
b. a second bonding roller (104) having a circumferential outer surface comprising a second flat surface (104b) and second bonding surface (104a), said second bonding surface comprising a plurality of protrusions (114), said protrusions (114) being arranged to fit within said grooves (110)
wherein the first and second bonding surfaces (102a,104a) are arranged to define the periphery of at least one absorbent article (10) and said first and second flat surfaces (102b, 104b) being arranged within the areas defined by the first and second bonding surface (102a, 104a) respectively.

8. Apparatus for bonding a topsheet to a backsheet according to claim 7, wherein the protrusions (114) are frusto-pyramidal shaped and are aligned in the machine direction (MD) in at least one column.

9. Apparatus according to claim 7 or 8 wherein said grooves (110) and ridges (112) are continuous in the machine direction (MD) when defining the periphery of one absorbent article (10).

10. Method suitable for manufacturing an absorbent article (10) according to claim 1 to 6, the method comprising:
- providing a topsheet (14) consisting essentially of natural fibers,
- providing a backsheet (16) comprising plastic material,
- arranging an absorbent core (12) comprising absorbent material in between said topsheet (14) and backsheet (16),
- applying a mechanical bonding with an apparatus according to claim 7 to 9, in said bonding region (13) along the periphery of the absorbent article (10) to bond said topsheet (14) to said backsheet (16), said bonding region (13) being substantially free of adhesive.

11. Method suitable for manufacturing an absorbent article (10) according to claim 1 to 6, the method comprising:
- providing a topsheet (14) consisting essentially of natural fibers,
- providing a backsheet (16) comprising plastic material,
- arranging an absorbent core (12) comprising absorbent material in between said topsheet (14) and backsheet (16),
- applying an ultrasonic bonding with an apparatus comprising:
a. an ultrasonic welding device comprising a sonotrode inducing vibrations at a frequency between 10 kHz and 50 kHz, preferably between 15 kHz and 30 kHz,
b. a second bonding roller (104) having a circumferential outer surface comprising a second flat surface (104b) and second bonding surface (104a), said second bonding surface comprising a plurality of protrusions (114),
wherein the second bonding surface (104a) is arranged to define the periphery of at least one absorbent article (10) and said second flat surfaces (104b) being arranged within the areas defined by the first and second bonding surface (104a) respectively, said ultrasonic bonding being applied in said bonding region (13) along the periphery of the absorbent article (10) to bond said topsheet (14) to said backsheet (16), said bonding region (13) being substantially free of adhesive.
